Europäisches Patentamt

European Patent Office

Office européen des brevets

(11) Publication number: **0 256 671 B1**

# EUROPEAN PATENT SPECIFICATION

(45) Date of publication of patent specification: **30.12.92**  (51) Int. Cl.⁵: **A61B 1/22**, A61B 17/36

(21) Application number: **87306260.8**

(22) Date of filing: **15.07.87**

(54) **Myringotomy instrument.**

(30) Priority: **25.07.86 US 890485**

(43) Date of publication of application:
**24.02.88 Bulletin 88/08**

(45) Publication of the grant of the patent:
**30.12.92 Bulletin 92/53**

(84) Designated Contracting States:
**DE FR GB**

(56) References cited:
**DE-B- 1 547 420**
**DE-B- 2 155 853**
**US-A- 4 141 362**
**US-A- 4 211 229**

(73) Proprietor: **Gabriel, Noble**
**13 Copley Drive**
**Andover Massachusetts 01810(US)**

(72) Inventor: **Gabriel, Noble**
**13 Copley Drive**
**Andover Massachusetts 01810(US)**

(74) Representative: **Ouest, Barry et al**
**M'CAW & Co. 41-51 Royal Exchange Cross**
**Street**
**Manchester M2 7BD(GB)**

Rank Xerox (UK) Business Services

## Description

The invention relates generally to otoscopes and the like, and more particularly to an instrument for performing myringotomies.

One of the preferred treatments for serous otitis media (SOM) is myringotomy, particularly in cases in which treatment with antibiotics have failed to overcome the underlying infection. One method of performing myringotomy is by incision with a knife, for example, as described by Dr. Richard H. Schwartz in "Myringtomy: A neglected Office Procedure", in AFP for December 1979. Recently there have been cases in which the myringotomy is performed by using a laser. See also the article in Pediatric Annals entitled "The Role of Myringotomy in Acute Otitis Media" by George A. Gates, dated May 5, 1984, p. 391; and an article by Luxford, et al, entitled "Myringotomy and Ventilation Tubes: A Report of 1,586 Ears" in "Laryngoscope 92, November, 1982, p. 1293. Recently it has been suggested that the myringotomy may be accomplished by the use of a laser. For example, as described by Richard L. Goode in Laryngoscope 92: April, 1982, p. 420; and also in a note in Journal of American Medical Association, v. 242, No. 88, August 24-31, 1979, p. 709; and in "Otological Applications of Lasers: Basic Background" by Chester Wilpizeski, et al. Further, "Myringotomy Made by $CO_2$ Laser -- an Alternative to the Ventilation Tube?", and experimental study by Solderberg, et al in Acta Otolaryngol (Stockh) 1984, 97: 335-341.

Also, of possible interest note the following U.S. Patents: 2,098,701 to Gagnon, November 9, 1937 for Diagnostic Instrument, disclosing a hand held otoscope with a light source for ear examination; 3,010,912 to Chester, February 13, 1962, for Motor Driven Surgical Knife which describes a motor driven knife for performing myringotomy.

Also, the following: U.S. Patent 3,865,114, February 11, 1974 to Sharon; for "Laser Device Particularly Useful as a Surgical Instrument" which describes a surgical laser with a mechanical shield to prevent excess radiation; U.S. Patent 3,982,541 to L'Esperance, Jr., September 28, 1976, for Eye Surgical Instrument which describes the use of a carbon dioxide ($CO_2$) laser for surgically removing surface portions of an eye, such as cataract tissue; United States Patent 4,006,738 to Moore, February 8, 1977, for Otoscope Construction which describes a halogen lamp for providing illumination for an otoscope; U.S. Patent 4,106,493 to Proctor, et al, August 15, 1978, for "Biphasic Otoscopic Air Stimulator for Performing Clinical Caloric Tests" describing an instrument and method of testing by bathing the tympanum and surrounding external tissue in fluid of predetermined temperature; U.S.

Patent 4,211,229 to Wurster, July 8, 1980, for Laser Endoscope, which describes the insertion of a laser and lens system in an endoscopic sheath; and U.S. Patent 4,366,811 to Riester, January 4, 1983, for Otoscope with Ejector Mechanism which describes an otoscope with a readily ejected ear funnel.

DE-A-1547420 describes an otoscope with a housing having a lens at a proximal end and a speculum at a distal end.

According to the invention, an otoscope and a laser beam generator are combined in a single instrument suitable for holding in the hand. The laser is rigidly connected to the otoscope. A reflector is provided arranged in the otoscope diagonally to the optical axis of the otoscope, and to the optical axis of the laser to reflect the laser beam along the line of sight of, and toward the distal end of the otoscope. Therefore, the operator or surgeon who is preparing to perform a myringotomy can view the operating field through the otoscope, and at the same time observe the effects of the laser beam surgery. The laser beam generator is adjustable in the tube in which it is held axially for adjusting the proper place of focus, rather than adjusting the focal length, thereby achieving a close adjustment.

By making the instrument hand-held, it may be used more accurately, and with the adjustment described with better control than manipulating separate instruments or adjusting focal length. The reflector may comprise ground and polished quartz, or a stainless steel element polished to a mirror surface. The otoscope may include means for providing a dispersed illumination of the viewing area, and if desired a suction tube for withdrawing exudate, or the like.

The various objects, advantages and novel features of the invention will be more fully understood from a reading of the following detailed description when read in connection with the accompanying drawing, in which like reference numerals refer to like parts, and in which:

Fig. 1 is a central longitudinal cross-sectional view, partly schematic, of the combined otoscope and laser instrument of the invention;

Fig. 2 is a partial sectional view, slightly enlarged, of a detail of Fig. 1, illustrating the adjustment means; and

Fig. 3 is an alternative to means of Fig. 1 for providing diffuse illumination for the otoscope of Fig. 1.

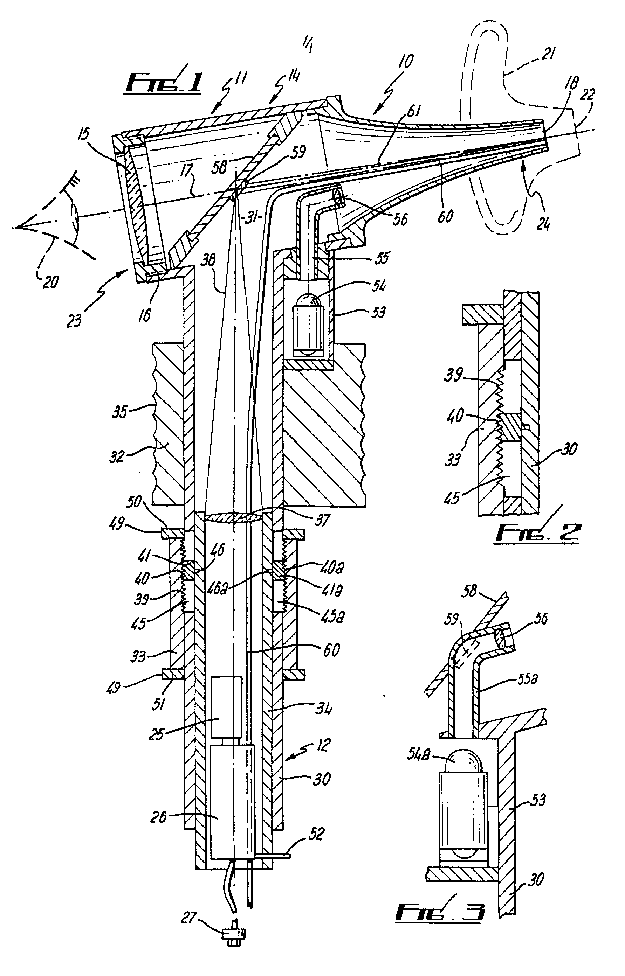

Referring to Fig. 1, the myringotomy instrument 10 comprises an otoscope 11, and a laser beam generator section 12, which may be a carbon dioxide ($CO_2$) laser or other, for example, an Argon laser. The otoscope comprises a housing 14, ocular lens at lens systems 15 in the housing 14, and a threaded adjustment 16 in the housing for moving the ocular lens 15 forward or back, in the direction of the optical axis 17 of the lens 15. The housing leads from the lens 15 in the proximal end 23 toward the distal end 24, where the housing terminates in a speculum, or opening, 18, which may, if desired, be removable by any suitable expedient, here shown as simply attached by a slight frictional and inherent spring attachment. At its distal end 24, the speculum terminates in an opening 18. Shown in dashed lines are the human eye 20 of the operator, and the ear lobe 21 of a patient as they might appear with the instrument in place. The tympanum of the ear which is to be the subject of the myringotomy is shown at 22.

The laser section 12 comprises a laser beam generator and power source indicated schematically at 26 to generate the laser beam which may be batteries within the tube, or a power converter for converting the usual ac electrical supply to the requisite laser supply, such as dc, and is therefore, supplied by the usual plug 27 for connection to any suitable outlet. A tube 30 contains the generator 26, and the tube 30 is rigidly connected to the otoscope housing 14. The housing 14 has an opening at 31 to receive the beam 38 from the laser generator 26. A mirror 25 may be provided for use with an external laser beam generator and/or a power source 47 and external laser beam 48.

A plastic hand grip 32 may surround and be attached to the tube 30, and furnished with external grooves 35 so that the hand may fit comfortably around the grip. The tube 30 carries an outer sleeve 33, and an inner sleeve 34. The inner sleeve 34 (see also Fig. 2) carries the generator 26 within the tube 30. A laser lens system indicated by the single lens 37 is carried also by the inner sleeve 34 as part of the laser generator 26. The outer sleeve 33 is internally threaded as at 39 to match and mesh with similar threads 40 and 40a on the outer surfaces of a pair of traveling elements 41 and 41a on diametrically opposite sides of tube 30. Although one element would be operable, the two opposite elements 40 and 40a tend to avoid possible canting of the inner sleeve 34, and thus enable smoother operation. The inner sleeve 34 slides inside the tube 30 in a close sliding fit. On diametrically opposite sides in tube 30, a pair of longitudinal, axial slots 45 and 45a receive respectively the traveling elements 41 and 41a. The elements 41 and 41a may slide only axially longitudinally in the slots 45 and 45a in which they fit closely. Consequently, when the outer sleeve 33 is rotated about the tube 30, the traveling elements 41 and 41a are forced to follow the slots longitudinally. The elements 41 and 41a have projections 46 and 46a inserted, such as by screwing into the inner tube 34. Therefore, as the traveling elements 41 and 41a travel axially, they carry the inner sleeve 34 axially with them. A pair of collars 49 having an upper collar 50, and a lower collar 51, respectively above and below the outer sleeve 33, restrain the outer sleeve 33 from axial motion and permit only rotational motion of the outer sleeve. A switch 52 may actuate the laser generator 26.

A housing 53 may be attached to the tube 30 adjacent the otoscope casing and carries a light source 54 which may supply light to an optical fiber bundle 55, through a lens 56. The bundle carries the light through an appropriate opening in the housing 14, such as at 18, and is faced so that light exiting the bundle is thrown as a diffuse light into the operating area, to enhance the viewing by the operator of the area of the myringotomy.

In the otoscope a strut 58 extending diagonally from top to bottom of the otoscope 14, carries a laser beam reflecting element 59 faced diagonally to intercept the laser beam 38 and deflect the beam toward the opening 18 of the otoscope so that the beam may be focused appropriately for the proposed myringotomy. The element 59 may be an optically ground and polished quartz or stainless steel with a polished reflecting surface.

A flexible suction tube 60 may lead from a suction apparatus into the interior of the otoscope housing through the opening 18, or may lead along the exterior of the otoscope to withdraw any fumes, exudate, or the like in the area.

In operation, the operator or surgeon applies the otoscope in the usual manner to the ear 21 of a patient so that he may view the tympanum 22. The light from the fibers 55 should be adequate to give the operator a view of the tympanum surface. The switch 52 is now actuated and preferably supplies an extremely short burst of the laser beam 38 which has a focal length determined by the lens system 37. The operator may judge from this initial burst which may be of microsecond duration, or the like, and the possible burn which it inflicts preferably without burning through the tissue, whether or not the focal length brings the laser beam onto the tympanum in a desired size, and at the correct place. He then can adjust the otoscope for placement, and adjust the lens system, as described, by rotating the outer sleeve 33 to advance or retard the focal point along the laser beam 61, (after the axis beam reflection) to afford the desired size of spot. By using very fine threads, the adjustment may be very close and fine. The operator may then repetitively actuate the switch to produce the short bursts until he achieves the desired opening in the tympanum.

If desired, a light source may be located adjacent the strut 58 on the tube 30 (as indicated in Fig. 3) as by a light source 54a with optical fiber bundle 55a facing the operating area. Alternatively, both sources 54 and 54a may be employed.

By making the threads 39 very fine, the motion of the lens system 37 may be adjusted very closely. Thus, rather than adjusting the focal length by moving one lens relative to another, the entire lens system is moved, giving a finer, more precise adjustment of the point along the beam axis at which the focal point of the laser beam occurs, and giving finer, more precise adjustment of the size of spot which may be burned by the beam, then by adjusting relative lens locations of the laser beam lens system.

**Claims**

1. A hand-held myringotomy instrument comprising:

an otoscope comprising a housing (14) having a proximal and a distal end, an optical lens system having an ocular lens (15) at the proximal end and having an optical axis (17), and a speculum (18) at the distal end, whereby an observer may view through the ocular lens from the proximal end along the optical axis a desired area near and beyond the distal end;
characterised by the provision of
a tube (30) rigidly connected to the housing (14);
a laser beam source (26) in the tube (30) for providing a laser beam along a beam path within the tube (30);
a laser lens system (37) in the tube (30) for focusing at a focal point the laser beam;
means (33, 34, 39-41) for moving along the laser beam axis the entire laser lens system (37) to move the laser beam focal point along the laser beam path;
a laser beam reflector (59) in the otoscope interposed diagonally in the laser beam path axis and diagonally in the optical axis (17) to redirect by reflection the laser beam coincidentally with the optical axis (17) toward the otoscope distal end, the laser lens system (37) focusing the laser beam at a point beyond the otoscope distal end;
whereby an operator may observe from the proximal end the area at which the laser beam is directed near the distal end of the otoscope and by said moving means (33, 34, 39-41) adjust along the laser beam axis the point of focus of the laser beam.

2. A myringotomy instrument as claimed in claim 1
characterised in that:
said means for moving the laser lens system (37) comprises an inner sleeve (34) carrying the laser lens system (37) inside the tube (30);

an outer sleeve (33) about the tube (30) internally threaded and restricted to rotation about the tube (30);
and a travelling element (41) fastened to the inner sleeve (34) and externally threaded to mesh with the outer sleeve (33) internal threads;
the tube (30) having a longitudinal slot (45) into which the element (41) projects and restricting the element (41) to axial motion along the tube (30), whereby rotation of the outer sleeve (33) moves the element (41) axially, and the attached inner sleeve (34) thereby moves the laser lens system (37) carrier axially in the inner sleeve (34).

3. A myringotomy instrument as claimed in claim 1
characterised in that:
said reflector (59) comprises a quartz plate having a polished reflecting surface to reflect the laser beam.

4. A myringotomy instrument as claimed in claim 1
characterised in that:
said reflector (59) comprises a steel plate having a polished reflecting surface to reflect the laser beam.

5. A myringotomy instrument as claimed in claim 2
characterised by the further provision of:
a second travelling element (41a) fastened to the inner sleeve (34) diametrically opposed to the first element (41) and externally threaded to mesh with the outer sleeve (33) internal threads;
the tube (30) having a second longitudinal slot (45a) diametrically opposed to the first slot (45) into which the second element (41a) projects and restricts the motion of the second element (41a) to axial motion along the tube (30), thereby promoting a balanced axial motion of the inner sleeve (34).

6. A myringotomy instrument as claimed in claim 1
characterised by the further provision of:
a light source (54), and an optical fiber bundle (55) placed in said otoscope to direct diffused light through the bundle (55) toward the distal end of the otoscope.

**Patentansprüche**

1. Handgeführtes Myringtomie-Instrument, umfassend: ein Otoskop mit einem Gehäuse (14), das ein proximales und ein distales Ende aufweist, ein optisches System, das eine Okularlinse (15) am proximalen Ende und eine optische Achse (17) aufweist, und ein Spekulum (18) am distalen Ende, so daß ein Beobachter durch die Okularlinse hindurch vom proximalen Ende entlang der optischen Achse einen gewünschten Bereich nahe und außerhalb des distalen Endes betrachten kann;
gekennzeichnet durch

ein Rohr (30), das starr mit dem Gehäuse (14) verbunden ist;

eine in dem Rohr (30) befindliche Laserstrahl-Quelle (26) zur Erzeugung eines Laserstrahls entlang einem Strahlengang innerhalb des Rohres (30);

ein dem Laser zugeordnetes optisches System (37) innerhalb des Rohres (30) zum Fokussieren des Laserstrahles auf einen Fokussierpunkt;

eine Einrichtung (33, 34, 39-41) zum Verstellen des gesamten dem Laser zugeordneten optischen Systems (37) entlang der Laserstrahl-Achse, um den Laserstrahl-Fokussierpunkt entlang dem Laser-Strahlengang zu bewegen; einen innerhalb des Otoskops befindlichen LaserstrahlReflektor (59), der diagonal in die Laserstrahlengang-Achse und diagonal in die optische Achse (17) eingefügt ist, um den Laserstrahl durch Reflexion in Koinzidenz mit der optischen Achse (17) zum distalen Ende des Otoskops zurückzulenken, wobei das dem Laser zugeordnete optische System (37) den Laserstrahl auf einen Punkt außerhalb des distalen Endes des Otoskops fokussiert;

so daß ein Benutzer des Instrumentes vom proximalen Ende aus den Bereich beobachten kann, auf den der Laserstrahl nahe dem distalen Ende des Otoskops gerichtet ist, und mittels der Verstelleinrichtung (33, 34, 39-41) den Fokussierpunkt des Laserstrahls entlang der Laserstrahlachse einstellen kann.

2. Myringtomie-Instrument nach Anspruch 1, dadurch gekennzeichnet, daß:
die Verstelleinrichtung für das dem Laser zugeordnete optische System (37) einen inneren Tubus (34) aufweist, der dieses optische System (37) innerhalb des Rohres (30) trägt;
ein das Rohr (30) umgebender äußerer Tubus (33) vorhanden ist, der ein Innengewinde aufweist und um das Rohr (30) lediglich drehbar ist;
ein Wanderelement (41) vorgesehen ist, das an dem inneren Tubus (34) befestigt ist und ein Außengewinde aufweist, das mit dem Innengewinde des äußeren Tubus (33) in Eingriff steht;

das Rohr (30) einen Längsschlitz (45) hat, in den das Element (41) eingreift, und der dem Element (41) lediglich eine axiale Bewegung entlang dem Rohr (30) erlaubt, so daß eine Drehbewegung des äußeren Tubus (33) das Element (41) axial verschiebt und der an dem Element angebrachte innere Tubus (34) dadurch das dem Laser zugeordnete, im inneren Tubus (34) abgestützte optische System (37) axial verschiebt.

3. Myringtomie-Instrument nach Anspruch 1, dadurch gekennzeichnet, daß:
der Reflektor (59) eine Quarzplatte aufweist, die eine polierte reflektierende Oberfläche zum Reflektieren des Laserstrahls hat.

4. Myringtomie-Instrument nach Anspruch 1, dadurch gekennzeichnet, daß:
der Reflektor (59) eine Stahlplatte aufweist, die eine polierte reflektierende Oberfläche zum Reflektieren des Laserstrahles hat.

5. Myringtomie-Instrument nach Anspruch 2, gekennzeichnet weiterhin durch:
ein zweites Wanderelement (41a), das an dem inneren Tubus (34) dem ersten Element (41) diametral gegenüberliegend angeordnet ist und ein Außengewinde aufweist, das mit dem Innengewinde des äußeren Tubus (33) in Eingriff steht;
das Rohr (30) hat einen zweiten Längsschlitz (45a), der dem ersten Schlitz (45) diametral gegenüberliegt, und in den das zweite Element (41a) eingreift, und der dem zweiten Element (41a) lediglich eine axiale Bewegung entlang dem Rohr (30) erlaubt, wodurch eine ausgeglichene Axialbewegung des inneren Tubus (34) gewährleistet ist.

6. Myringtomie-Instrument nach Anspruch 1, gekennzeichnet weiterhin durch:
eine Lichtquelle (54) und ein Lichtleitfaserbündel (55), das in dem Otoskop angeordnet ist, um diffuses Licht durch das Bündel (55) zum distalen Ende des Otoskops zu leiten.

**Revendications**

1. Instrument manuel de myringotomie comprenant :

un otoscope comportant un boîtier (14) ayant une extrémité distale et une extrémité proximale, un dispositif formant lentille optique ayant une lentille (15) formant oculaire située à l'extrémité proximale et ayant un axe optique (17), et un speculum (18) situé à l'extrémité distale, grâce à quoi un observateur peut, à travers la lentille formant oculaire de l'extrémité proximale, le long de l'axe optique, regarder une zone voulue située à proximité et au-delà de l'extrémité distale ;

caractérisé en ce qu'il comporte :

un tube (30) relié rigidement au boîtier (14) ;

une source (26) de rayon laser située dans le tube (30) pour émettre un rayon laser le long d'un trajet de rayon situé dans le tube (30) ;

un dispositif (37) formant lentille laser situé dans le tube (30) pour focaliser en un point focal le rayon laser ;

des moyens (33, 34, 39 - 41) destinés à déplacer la totalité du dispositif (37) formant lentille laser le long de l'axe du rayon laser pour déplacer le point focal du rayon laser le long du trajet du rayon laser ;

un réflecteur (59) de rayon laser situé dans l'otoscope et interposé diagonalement sur l'axe du trajet du rayon laser et diagonalement sur l'axe optique (17) pour diriger le rayon laser, par réflexion, vers l'extrémité distale de l'otoscope en coïncidence avec l'axe optique (17), le dispositif (37) formant lentille laser focalisant le rayon laser en un point situé au-delà de l'extrémité distale de l'otoscope ;

grâce à quoi un opérateur peut observer à partir de l'extrémité proximale la zone sur laquelle le rayon laser est dirigé, située à proximité de l'extrémité distale de l'otoscope, et en ce que les moyens de déplacement (33, 34, 39 - 41) règlent le point de focalisation du rayon laser le long de l'axe du rayon laser.

2. Instrument de myringotomie selon la revendication 1, caractérisé en ce que :

les moyens de déplacement du dispositif (37) formant lentille laser comportent un manchon intérieur (34) portant le dispositif (37) formant lentille laser, situé à l'intérieur du tube (30) ;

un manchon extérieur (33) entourant le tube (30), fileté intérieurement et empêché de tourner par rapport au tube (30) ;

et un élément mobile (41) fixé sur le manchon intérieur (34) et fileté extérieurement pour être en prise avec le manchon extérieur (33) fileté intérieurement ;

le tube (30) ayant une fente longitudinale (45) dans laquelle l'élément (41) fait saillie, et limitant le déplacement axial de l'élément (41) le long du tube (30), grâce à quoi la rotation du manchon extérieur (33) déplace axialement l'élément (41), et le manchon intérieur (34) qui y est fixé déplaçant ainsi le support de dispositif (37) formant lentille laser, axialement dans le manchon intérieur (34).

3. Instrument de myringotomie selon la revendication 1, caractérisé en ce que :

le réflecteur (59) comporte une plaque de quartz ayant une surface réfléchissante polie destinée à réfléchir le rayon laser.

4. Instrument de myringotomie selon la revendication 1, caractérisé en ce que :

le réflecteur (59) comporte une plaque d'acier ayant une surface réfléchissante polie destinée à réfléchir le rayon laser.

5. Instrument de myringotomie selon la revendication 2, caractérisé en ce qu'il comporte en outre :

un second élément mobile (41a) fixé sur le manchon intérieur (34), diamétralement opposé au premier élément (41) et fileté extérieurement pour être en prise avec le manchon extérieur (33) fileté intérieurement ;

le tube (30) ayant une seconde fente longitudinale (45a) diamétralement opposée à la première fente (45), dans laquelle le second élément (41a) fait saillie et limitant le déplacement axial du second élément (41a) le long du tube (30), favorisant ainsi un mouvement axial équilibré du manchon intérieur (34).

6. Instrument de myringotomie selon la revendication 1, caractérisé en ce qu'il comporte en outre :

une source lumineuse (54), et un faisceau (55) de fibres optiques situé dans l'otoscope pour diriger la lumière diffusée à travers le faisceau (55) vers l'extrémité distale de l'otoscope.

FIG. 1

FIG. 2

FIG. 3